# EUROPEAN PATENT SPECIFICATION

(11) **EP 1 874 187 B1**
(45) Date of publication and mention of the grant of the patent: **11.03.2009**
(21) Application number: 06727692.3
(22) Date of filing: 21.03.2006
(51) Int. Cl.: A61B 5/103

(54) **DEVICE AND METHOD FOR AUTOMATICALLY TESTING THE TEMPERATURE SENSITIVITY OF A PATIENT**
VORRICHTUNG UND VERFAHREN ZUM AUTOMATISCHEN PRÜFEN DER TEMPERATUREMPFINDLICHKEIT EINES PATIENTEN
PROCEDE ET DISPOSITIF D'ESSAI AUTOMATIQUE DE LA SENSIBILITE A LA TEMPERATURE D'UN PATIENT

(30) Priority: 22.03.2005 EP 05102287
(43) Date of publication of application: 09.01.2008
(73) Proprietor: Philips Intellectual Property & Standards GmbH, 20099 Hamburg (DE); Koninklijke Philips Electronics N.V., 5621 BA Eindhoven (NL)
(72) Inventor: LANFERMANN, Gerd, Philips IP & Standards GmbH, 52066 Aachen (DE); KELLNER, Andreas, Philips IP & Standards GmbH, 52066 Aachen (DE); BRAUERS, Andreas, Philips IP & Standards GmbH, 52066 Aachen (DE)
(74) Representative: Schouten, Marcus Maria
(86) International application number: PCT/IB2006/050860
(87) International publication number: WO 2006/100638

(56) References cited:
- EP-A- 0 242 814
- US-A- 5 191 896
- US-A- 5 381 805
- US-A1- 2004 243 021

## Description

The present invention relates to a device and method for automatically testing the temperature sensitivity of a patient. Furthermore the invention relates to a computer program for automatically testing the temperature sensitivity of a patient.

Stroke is caused by obstructed blood vessels or bleeding in the brain and leads to an undersupply of oxygen to the brain, followed by the decay of brain tissue. Stroke is a significant cause of death and disability. Early detection and treatment of strokes is essential to restore the neurological capability in an underperfused brain.

Often, the following typical stroke symptoms affect only one side of the body: muscle weakness, paralysis, numbness, vision problems. While a number of known stroke test devices examine the motor capabilities of the user through active procedures, no proper method exists to test for numbness, in particular in a home environment, where most strokes occur.
However, checking the neurological operation is important and conducted routinely as part of evoked potential tests, where the patient is stimulated by an image, a pinch and a sound while an EEG is measured.

Several prior art documents describe techniques for testing the temperature sensitivity of a patient in a hospital environment. Thermal tests are described e.g. in US 5 381 805, EP 0 242 814 A, and US 5 191 896 A. In all cases an operator is needed, either to instruct the patient, or to start the test procedure and the modify the test parameter.

It is an object of the present invention to complete existing test methods by providing a simple technique to test for numbness by the user himself.

This object is achieved according to the invention by a device for automatically testing the temperature sensitivity of a patient, the device comprising a number of contact areas, each area being arranged to get in contact with an area of the patient's body, and further adapted to cause a temperature stimulation of that body area, the device further comprising a response unit adapted to receive a response of the patient with respect to the patient's sensation and further adapted to generate a response signal accordingly, the device further comprising a control unit adapted to automatically control the sensitivity test depending on the response signal, and the device further comprising a number of monitoring means adapted to monitor whether the patient performs the test properly and further adapted to generate a compliance factor which may be used by the control unit.

The object of the present invention is also achieved by a method for automatically testing the temperature sensitivity of a patient, the method comprising the steps of: providing a number of contact areas being adapted to cause a temperature stimulation of a body area of the patient, starting the sensitivity test, receiving a response of the patient with respect to the patient's sensation, generating a response signal according to the patient's response, automatically controlling the sensitivity test depending on the response signal, and monitoring whether the patient performs the test properly and generating a compliance factor.

The object of the present invention is also achieved by a computer program for automatically testing the temperature sensitivity of a patient, the computer program comprising computer program instructions to generate a response signal according to the patient's response to a temperature stimulation of a body area of the patient, computer program instructions to automatically control the sensitivity test depending on the response signal, and computer program instructions to generate a compliance factor indicating whether the patient performs the test properly, when the computer program is executed in a computer. The technical effects necessary according to the invention can thus be realized on the basis of the instructions of the computer program in accordance with the invention. Such a computer program can be stored on a carrier or it can be available over the internet or another computer network. Prior to executing the computer program is loaded into a computer by reading the computer program from the carrier, for example by means of a CD-ROM player, or from the internet, and storing it in the memory of the computer. The computer includes inter alia a central processor unit (CPU), a bus system, memory means, e.g. RAM or ROM etc. and input/output units.

A basic idea of the present invention is to provide a technique, which enables a test for numbness for a patient in a home environment. The technique can be realized using a portable device, which can easily be handled by a single person. The main advantage of the invention is that the test can be performed without an operator, e.g. physician or nurse, simply by the patient on its own. The inventive technique allows a fully automated test procedure. By means of the response unit a response signal is generated. The response signal dose not only indicate the quality of the patient's responses (e.g. if the patient gives his response fast or slow). At the same time the response signal is used as "compliance factor", indicating whether or not the patient is actually performing the test at all and further indicating whether or not the patient is performing the test properly. In other words a feedback of the patient is received and used, according to the invention, to ensure a reliable test performance. The feedback is given directly to the test device and the feedback results are analysed automatically. Thus no operator is needed. Furthermore the test itself can performed automatically, thereby being fully controlled based on the feedback results.

These and other aspects of the invention will be further elaborated on the basis of the following embodiments, which are defined in the dependent claims.

According to a preferred embodiment of the invention contact areas are heat conducting in order to cause a temperature stimulation of the patient's body area. Preferably the contact areas are heated and/or cooled by means of one or more heating rods and/or Peltier elements, respectively. If the contact areas are pressure sensitive, the patient's contact to the contact area can be controlled and/or the test results can be corrected by a factor, which is dependent on the force of the patient's body area on the contact area. If the user applies variable pressure over time, the factor may depend on the time intervals and pressure strength, when a certain pressure was applied.

In a preferred embodiment of the present invention the number of contact areas are parts of a portable handheld interface. Preferably the contact areas are integrated into a stroke test device adapted for carrying out a motor test or the like. The contact areas are preferably arranged such that the patient inevitable touches the contact areas, when he takes hold of the device using one or both of his hands. In another embodiment the device comprises an inertia sensor to detect whether the device is actually moved and lifted up by the patient e.g. during a motor test. This can be used to automatically initiate a testing sequence through a verbal welcome message to the patient.

Alternatively the contact areas are integrated into a weighing machine. In this case the patient can perform the test during a routine weight measurement. In both cases the patient does not have to use connectors, cables, or the like, which greatly improves the manageability of the new technique. However, the device according to the present invention does not have to be part of a motor test device or a weighing machine. The inventive device may as well be designed as a stand-alone unit.

The feedback of the patient is received by the response unit, which preferably comprises a number of actuating elements, e.g. buttons, switches, or the like. If the contact area is adapted to serve as actuating element, no additional component is needed and in case of a handheld device the patient does not have to put his hands off the contact area for giving the feedback. In still another embodiment of the invention the actuating elements or other parts of the device are used as contact areas.

Alternatively or additionally a number of non-contact receiving elements can be provided. These non-contact receiving elements may be part of a voice interface, e.g. a microphone/speaker combination and/or a visual interface, e.g. a camera/monitor combination. The use of a non-contact receiving element is especially useful, if the patient cannot use his hands for giving feedback. Speech-impaired patients can preferably use the visual interface for giving feedback by nodding or shaking. In those cases the test device comprises means for carrying out voice and/or optical recognition.

In still another embodiment of the invention a camera and an according recognition means are used to monitor the patient as he is carrying out the test. For example it can be controlled, whether the patient looks at the monitor for receiving test instructions. Using an according control system the patient may be instructed automatically to look at the device and concentrate.

In a further embodiment of the invention the response unit is adapted to generate a response signal corresponding to the fact, whether or not the patient provides a response within a defined time period. If the sensitivity test is performed, e.g. during or after the execution of the motor tests, the contact areas are heated or cooled in a defined manner. Thereby the contact areas can be heated or cooled in different ways. E.g. a first contact area (e.g. the contact area situated to the left hand side of the handheld device) can be held at a constant temperature, whereas a second contact area (the contact area situated to the right hand side of the handheld device) is heated or cooled. In this case the patient may report, whether he can feel a temperature difference between the first and second contact area and he can indicate the quality of the temperature change ("warmer" or "cooler"). In another test the temperature of the left or the right contact area can be heated or cooled or both contact areas can be jointly heated or cooled. In this case the patient may report, whether he can feel a temperature change at one or both contact areas respectively and can indicate the quality of the temperature change.

If the patient experiences any temperature changes, he should report his sensation to the response unit, e.g. through the visual interface. If the response unit does not receive a response within the defined time period the patient either did not perform the test properly or did not sense a temperature change. To obtain a reliable test result, the part of the test the patient was not responding to is either repeated or an additional measure is used in order to assure the patient is taking part in the test, e.g. using a camera to check the patient's position. If the patient is not responding within the time period during the rerun of the test or if it can be assured that the patient actually was performing the test properly, a response signal is generated indicating that the patient has failed to respond in time.

In still another embodiment the response unit is adapted to generate a response signal corresponding to the patient's response time. In other words, if the patient responses to the temperature change, a response signal is generated, indicating the duration of the response time. All response signals are preferably stored and/or processes by the control unit.

In another embodiment of the invention the control unit is adapted to automatically generate and/or amend the sensitivity test or a certain part of the sensitivity test, depending on the response signal. In other words, the control unit uses the response, signals to automatically generate a complete sensitivity test or parts of the test. Preferably the next part of the sensitivity test is generated by the control system depending on the patient's feedback. E.g. the kind of test is changed from temperature difference test to temperature change test. If e.g. the course of test runs is defined and cannot be changed, the test can also be amended depending on the feedback results by means of the control unit. For example if the patient does not sense a certain temperature difference or temperature change in a test, the test row can be continued with a new generated test using another temperature characteristic. There are many possible ways to generate and amend sensitivity tests and not all variants can be described here. Preferably the control unit uses other data as well in order to define the testing procedure, e.g. historic data of the same or other patients, medical information, statistics etc. For generating or amending the test the control unit preferably uses a neuronal network or another intelligent data base system, which can be implemented as internal or external part of the device.

In another preferred embodiment of the invention the control unit is adapted to generate a test result based upon the response signals. The test result may by used (preferably together with other measuring results, e.g. the results of the motor test) to indicate the likelihood of a stroke to be occurred. In a preferred embodiment the rest results is used to generate a stroke indicator.

In another embodiment calibration data, which has been collected during an earlier sensitivity test, is used by the control unit for generating or amending the test or parts of the test or for generating the test result. For this purpose the control unit is adapted to store calibration data and/or to process or use calibration data. Calibrating is preferably carried out with respect to the patient's "normal" (i.e. healthy) status. For obtaining calibration data the patient preferably performs a full run of the sensitivity test and the response data (e.g. temperatures and response times) is recorded and stored for later use by the control unit. This reference data is preferably updated or refreshed at any time the test is running, provided the patient is still in a normal condition.

In another preferred embodiment of the invention the control unit is adapted to communicate with the patient. In other words, not only a one-way communication from the patient to the device is provided, but a two-way communication path is established between patient and test device. This communication is used preferably to question the patient about the patient's sensation. For example the patient can be asked, if he feels a temperature difference between the right and the left contact area or the patient can be asked, if he feels a change in temperature at one or both of the contact areas.

Especially in home use without any further human assistance the patient may feel insufficiently advised. In this case the control unit (in connection with the response unit, where applicable) informs inform the patient about the sensitivity test to be performed or being performed. For example the control unit can be adapted to give advise and/or instructions to handle the device or to perform the test. Of course, the control unit may inform the patient about the test result or even the stroke indicator. However, in a preferred embodiment the control unit is connectable to an external communication line, e.g. a telephone network or computer network in order to send a signal to the patient's physician, if the stroke indicator indicates a critical patient condition.

The monitoring means mentioned above, e.g. the camera, the inertia sensor etc., are preferably adapted to monitor the compliance of the patient. In a preferred embodiment of the present invention the monitoring results are used to generate a compliance factor, e.g. by the control unit. The control unit uses the compliance factor to rate or to weight a test sequence. For example the control unit may to invalidate a test sequence, if the compliance factor indicates that the patient was not performing the test properly. The control unit may also add a confidence measure to the test results, if the compliance factor indicates that the patient was performing the test as required.

In still another embodiment of the invention the device comprises a sensor unit to receive information on the ambient condition, particularly ambient temperature, time-of-day, etc. Using this information as external parameters, a nonlinear relationship for temperature increase may be defined for test iterations. Additionally the initial temperature can be set up depending on those external parameters.

The present invention is not only applicable with respect to neurological deficits caused by stroke. The invention may also be used in case of neurological deficits caused by other diseases, e.g. diabetes and Parkinson's Disease.

These and other aspects of the invention will be described in detail hereinafter, by way of example, with reference to the following embodiments and the accompanying drawings; in which:
Fig. 1 is a schematic block diagram of a device for automatically testing the temperature sensitivity of a patient,
Fig. 2 is a simplified front view of such a device,
Fig. 3 is a simplified rear view of such a device,
Fig. 4 is a flow chart illustrating a mode of operation of a such a device, and

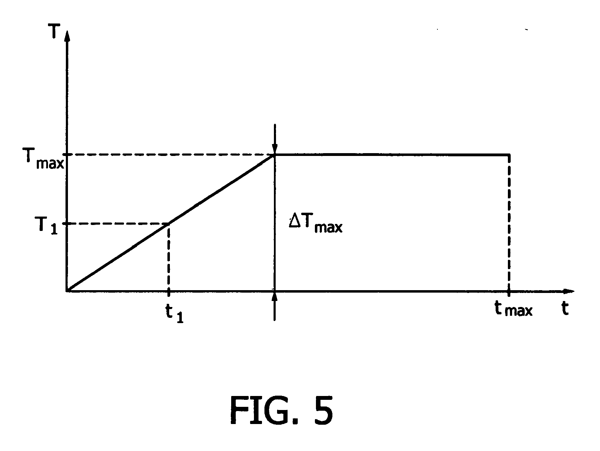

Fig. 5 is a diagram illustrating the temperature curve during a test run.

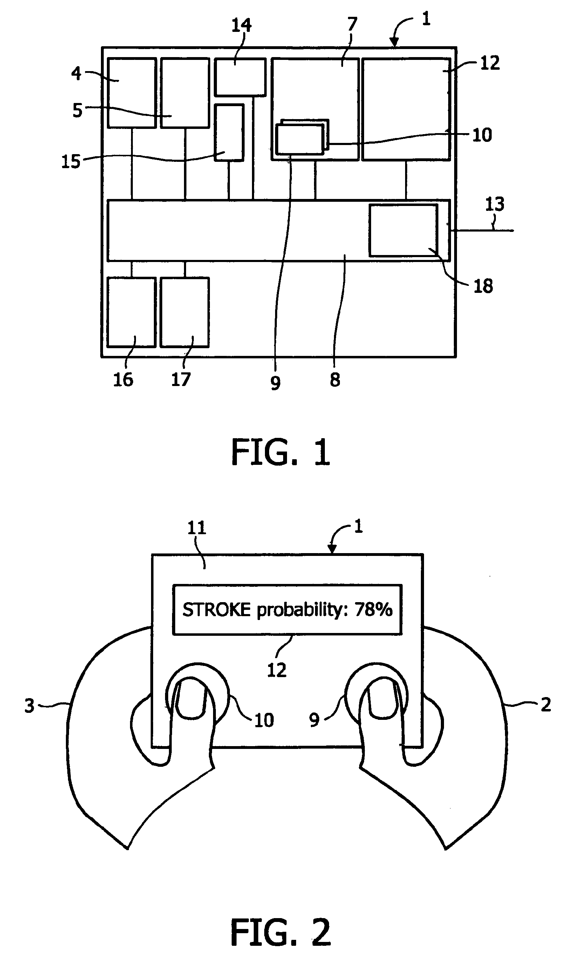

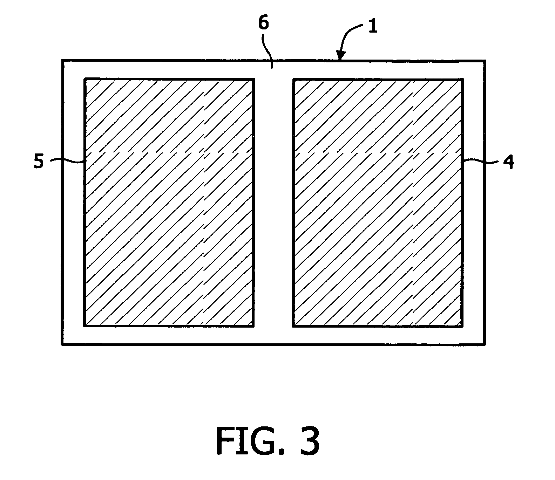

Figs. 1 to 3 show a device 1 for automatically testing the temperature sensitivity of a patient's fingers and/or hands 2, 3 and to detect and quantify any deficiencies in the sensitivity. The device is integrated into portable handheld interface of a stroke test device, as it is known for performing motor tests. The patient executes the standard motor experiments as part of the stroke test, e.g. pressing buttons, answering questions about images etc. While he is engaged in this process, the areas under his left and right hand 3, 2 are heated and/or cooled respectively. If the patient fails to respond with the correct answer, the result of the temperature sensitivity test is added to the evaluation of the stroke test as an additional stroke indicator.

For implementing the invention the handheld interface comprises two contact areas 4, 5 at the rear side 6 of the device 1. Each contact area 4, 5 is arranged such, that a patient holding the device 1 using his hands 2, 3 will get in contact with the two contact areas 4, 5. The contact areas 4, 5 are positioned on the back of the device 1, where the hands 2, 3 are rested. The contact areas 4, 5 are adapted to cause a temperature stimulation of that patient's hands 2, 3. For this purpose both contact areas 4, 5 are made of heat conducting material and can be heated and/or cooled by means of heating rods and/or Peltier elements, which are not shown in detail. The contact areas 4, 5 are pressure sensitive, thus the patient's contact to the contact areas 4, 5 can be controlled, as explained later.

The device 1 further comprises a response unit 7 and a control unit 8. The response unit 7 is adapted to receive a response of the patient with respect to the patient's sensation. For this purpose the response unit 7 comprises two button 9, 10 on the front side 11 of the handheld device 1, i.e. opposite the contact areas 4, 5 on the rear side 6. The two buttons 9, 10 may already be part of the stroke test device and used for examination of the coordination abilities of the patient. In this case those buttons is given an additional functionality by the present invention. Buttons 9, 10 and contact areas 4, 5 are arranged in a way that the left button 10 receives the patient's feedback (i.e. the patient is pressing the button) in case the patient senses a temperature change of the left contact area 5 and the right button 9 receives the patient's feedback in case the patient senses a temperature change of the right contact area 4. If a test is performed, wherein the sensing of a temperature difference between the two contact areas 4, 5 is tested, either one or both buttons 9, 10 may be used by the patient for give a feedback to the test device 1.

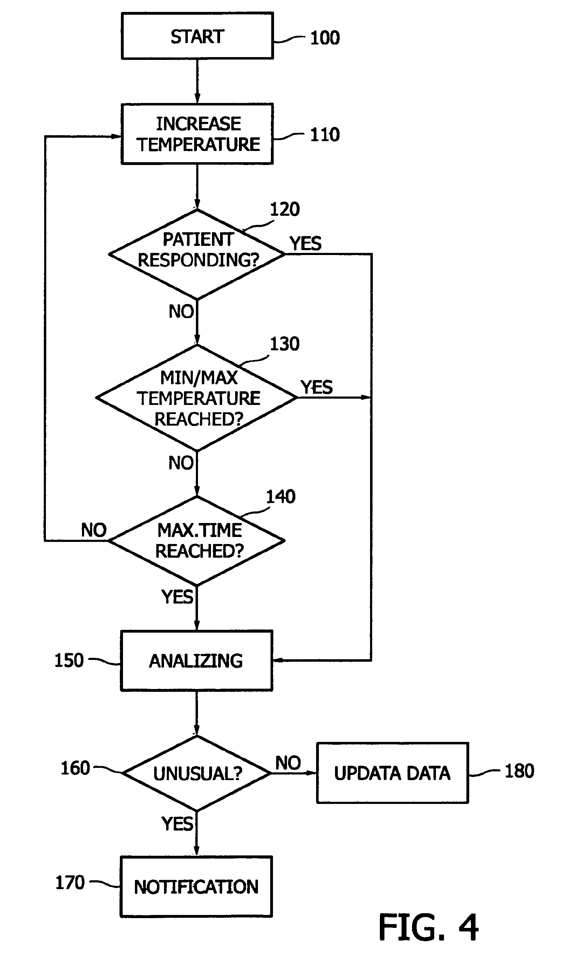

The response unit 7 is further adapted to generate a response signal according to the patient's feedback. A single test will now be explained with reference to Fig. 4. After switching on the device 1 by the patient and displaying a welcome message to the patient on the front side monitor 12 of the device 1 the test starts in step 100. At the start of the test, the temperature of the contact area may be either held at ambient temperature or preheated/cooled to a well-defined temperature, which may be fixed or chosen depending on parameters like, ambient temperature, time-of-day, etc. Subsequently the temperature of the left contact area 5 is increased by a temperature increase AT of 3°C in step 110. The heating (orcooling) of the contact area 5 may be carried out with or without an announcement by the device. Now the response unit 7 waits for the patient pressing the left button 10 in step 120. If the patient is responding, the response time t₁ and the value of the temperature T₁(t₁) is analyzed in the control unit 8 in step 150 as explained below. The temperature increase is stopped when the patient gives a response or when the maximum temperature Tₘₐₓ is reached (step 130). If no patient response is detected the test continues until the maximum allowed response time tₘₐₓ is expired (step 140). In this case, the currently applied temperature is increased by ΔT and the previously described steps are repeated. ΔT may be different in each loop, allowing for fine temperature resolution in the temperature range, where a normal user is expected to perceive temperature changes. The ΔT may be increased (e.g. doubled) if it becomes clear that the user fails to respond to obvious temperature changes. This allows for quick coverage of the full temperature range up to T_{max.} The ΔT-per-step-relationship may be linear, exponential or otherwise prescribed. It may depend on past user performance or depend on the initial temperature. A temperature sensor (not shown) will detect ambient temperature.

In all cases the response unit 7 generates a response signal R corresponding to the fact, whether or not the patient provides a response within a defined time period or corresponding to the patient's response time accordingly. For example, if the patient did not press the left button 10 during the test, a response signal R=(AT, Tₘₐₓ, tₘₐₓ, 0) is generated. If the patient pressed the button 10 after a response time t₁ a response signal R=(ΔT, Tₘₐₓ, tₘₐₓ, t₁, T₁) is generated. All response signals are stored within the control unit 8.

The control unit 8 is adapted to automatically control the sensitivity test depending on the response signal R. In other words the test results are analyzed (step 160) and the next step is carried out depending on the outcome of the analysis. For this purpose the test results are compared with historic test results (reference data), which has been obtained in one or more earlier tests, and a trend analysis is curried out. Prior to analysis the device 1 is calibrated with respect to the reference data, i.e. with respect to proper functioning extremities. During analyzing the test results the test results are corrected by the control unit 8 depending on the force of the patient's hands on the pressure sensitive contact area. If the analysis shows that the patient has failed to report a temperature change, there is a strong indication for a neural disorder in the respective limb.

In the illustrated embodiment the following options are provided: If, for example, the temperature difference ΔT equals the maximum allowed temperature difference ΔTₘₐₓ without reaction by the patient, the patient is notified accordingly in the next step 170. If the response time t₁ exceeds the maximum allowed response time tₘₐₓ, the patient is notified accordingly in step 170. If the response time t₁ is above the average historic response time (i.e. "button pressed too late"), the patient is notified accordingly in step 170. If the response time t₁ is below the average, historic button press duration (i.e. "button pressed too early"), the test is repeated after a refractory period. In this period, the contact areas 4, 5 are reset to initial temperature and the hands 2, 3 (or fingers) are given time to accommodate to the new temperature. During this time, the user is either entertained or asked to perform other tests. If the test results are still persistent during the second test and the patient is healthy, the reference data is updated in step 180.

The notification of the patient is performed using the monitor 12 of the handheld device 1. Thereby different types of information may be provided to the patient. In a simple version only a test result in form of a "PASSED" or "FAILED" message is displayed. In another version one ore more of the actual test results ΔT, Tₘₐₓ, tₘₐₓ, t₁, T₁ are provided. In still another version a stroke indicator generated by the control unit 8 is displayed, e.g. in the form of "STROKE PROBABILITY: 78%". The control unit 8 is connectable to an external communication line 13 in order to send a signal to the patient's physician, if the test results indicate a critical patient condition. Instead of notifying the patient and/or sending a signal via the external communication line 13, other actions may be taken.

The control unit 8 does not only control the built-in monitor 12. Additionally an internal communication interface is controlled, which is adapted to provide instructions to handle the device 1 or to perform the test. The interface comprises a microphone 14 and a speaker 15 and is part of the handheld device 1, see Fig. 1. Using the speaker 15, the control unit 8 e.g. indicates to the patient during step 110 that the test has started and the patient should give a feedback by pressing the left button 10, when sensing a temperature change of the left contact area 5. In other words, the control unit 8 serves as an operator guiding the patient through the test procedure. Such guidance increases the acceptance of the test device 1. To avoid guessing, the control unit 8 can even pose the question, whether or not the patient senses a temperature change, though the contact areas 4,5 are not heated or cooled. In another version the patient is asked to perform certain motor or other tests until he experiences a change in temperature on the left or right hand 3, 2. The resulting timing data is stored in the control unit 8 and compared for long-term evaluation by the control unit 8.

The device 1 further comprises monitoring means, namely a video camera 16 and an inertia sensor 17, to monitor the compliance of the patient. The obtained data are analyzed by the control unit 8 and used for generating the test results. Thereby the impact of the monitored data on the test results depends on the compliance of the patient. If the patient does not carry out the test properly, e.g. if he presses the button in an irregular way, the control unit 8 may invalidate the corresponding test sequence.

All data processing involved in the sensitivity test as described above, particularly the generating of the response signal according to the patient's response and the controlling of the test depending on the response signal, is carried out using a computer 18 in which a computer program according to the present invention is executed. Preferably the control unit 8 comprises such a computer 18.

It will be evident to those skilled in the art that the invention is not limited to the details of the foregoing illustrative embodiments, and that the present invention may be embodied in other specific forms without departing from the essential attributes thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. It will furthermore be evident that the word "comprising" does not exclude other elements or steps, that the words "a" or "an" do not exclude a plurality, and that a single element, such as a computer system or another unit may fulfil the functions of several means recited in the claims. Any reference signs in the claims shall not be construed as limiting the claim concerned.

### REFERENCE LIST

- 1: test device
- 2: patient's right hand
- 3: patient's left hand
- 4: right contact area
- 5: left contact area
- 6: rear side
- 7: response unit
- 8: control unit
- 9: right button
- 10: left button
- 11: front side
- 12: monitor
- 13: communication line
- 14: microphone
- 15: speaker
- 16: video camera
- 17: inertia sensor
- 18: computer

## Claims

1. A device (1) for automatically testing the temperature sensitivity of a patient, comprising
- a number of contact areas (4, 5), each area being arranged to get in contact with an area (2, 3) of the patient's body, and further adapted to cause a temperature stimulation of that body area (2, 3),
- a response unit (7) adapted to receive a response of the patient with respect to the patient's sensation and further adapted to generate a response signal accordingly,
- a control unit (8) adapted to automatically control the sensitivity test depending on the response signal, and **characterized by**
a number of monitoring means (16, 17) adapted to monitor whether the patient performs the test properly and further adapted to generate a compliance factor which may be used by the control unit (8).

2. The device (1) as claimed in claim 1, wherein the number of contact areas (4, 5) are heat conducting and/or pressure sensitive.

3. The device (1) as claimed in claim 1, wherein the number of contact areas (4, 5) are parts of a weighing machine.

4. The device (1) as claimed in claim 1, wherein the response unit comprises a number of actuating elements (9, 10) and/or a number of non-contact receiving elements (16).

5. The device (1) as claimed in claim 1, wherein the response unit (7) is adapted to generate a response signal corresponding to the fact, whether or not the patient provides a response within a defined time period and/or corresponding to the patient's response time.

6. The device (1) as claimed in claim 5, wherein the control unit (8) is adapted to automatically generate and/or amend the sensitivity test or a certain part of the sensitivity test, depending on the response signal.

7. The device (1) as claimed in claim 1, wherein the control unit (8) is adapted to store calibration data and/or to process or use calibration data during the sensitivity test and/or during generation of the test result, said calibration data being provided during an earlier sensitivity test

8. A method for automatically testing the temperature sensitivity of a patient, comprising the steps of
- providing a number of contact areas (4, 5) being adapted to cause a temperature stimulation of a body area (2, 3) of the patient,
- starting the sensitivity test,
- receiving a response of the patient with respect to the patient's sensation,
- generating a response signal according to the patient's response,
- automatically controlling the sensitivity test depending on the response signal, and
- monitoring whether the patient performs the test properly and generating a compliance factor.

9. The method as claimed in claim 8, comprising the steps of automatically generating and/or amending the sensitivity test or a certain part of the sensitivity test, depending on the response signal.

10. The method as claimed in claim 8, comprising the step of generating a test result based upon the response signals.

11. The method as claimed in claim 8, comprising the step of storing calibration data and/or processing or using calibration data during the sensitivity test and/or during generation of the test result, said calibration data being provided during an earlier sensitivity test.

12. A computer program for automatically testing the temperature sensitivity of a patient, comprising
- computer program instructions to generate a response signal according to the patient's response to a temperature stimulation of a body area (2, 3) of the patient,
- computer program instructions to automatically control the sensitivity test depending on the response signal, and
- computer program instructions to generate a compliance factor indicating whether the patient performs the test properly,
when the computer program is executed in a computer (18).

## Patentansprüche

1. Vorrichtung (1) zum automatischen Prüfen der Temperaturempfindlichkeit eines Patienten, mit
- einer Anzahl von Kontaktflächen (4, 5), wobei jede Fläche angeordnet ist, um in Kontakt mit einer Fläche (2, 3) des Patientenkörpers zu kommen, und weiterhin dafür eingerichtet ist, eine Temperaturstimulation dieser Körperfläche (2, 3) zu verursachen,
- einer Reaktionseinheit (7), die dafür eingerichtet ist, eine Reaktion des Patienten in Bezug auf die Empfindung des Patienten zu empfangen, und weiterhin dafür eingerichtet ist, ein entsprechendes Reaktionssignal zu erzeugen,
- einer Steuereinheit (8), die dafür eingerichtet ist, die Empfindlichkeitsprüfung automatisch in Abhängigkeit von dem Reaktionssignal zu steuern, und **gekennzeichnet durch**
eine Anzahl von Überwachungsmitteln (16, 17), die dafür eingerichtet sind, zu überwachen, ob der Patient die Prüfung ordnungsgemäß durchführt, und weiterhin dafür eingerichtet sind, einen Einhaltungsfaktor (engl. compliance factor) zu erzeugen, der **durch** die Steuereinheit (8) verwendet werden kann.

2. Vorrichtung (1) nach Anspruch 1, wobei die Anzahl der Kontaktflächen (4, 5) wärmeleitend und/oder druckempfindlich sind.

3. Vorrichtung (1) nach Anspruch 1, wobei die Anzahl der Kontaktflächen (4, 5) Teile einer Wiegemaschine sind.

4. Vorrichtung (1) nach Anspruch 1, wobei die Reaktionseinheit eine Anzahl von Betätigungselementen (9, 10) und/oder eine Anzahl von berührungslosen Empfangselementen (16) umfasst.

5. Vorrichtung (1) nach Anspruch 1, wobei die Reaktionseinheit (7) dafür eingerichtet ist, ein Reaktionssignal zu erzeugen, das der Tatsache entspricht, ob der Patient innerhalb einer definierten Zeitspanne eine Reaktion liefert oder nicht und/oder das der Reaktionszeit des Patienten entspricht.

6. Vorrichtung (1) nach Anspruch 5, wobei die Steuereinheit (8) dafür eingerichtet ist, automatisch in Abhängigkeit vom Reaktionssignal die Empfindlichkeitsprüfung oder einen bestimmten Teil der Empfindlichkeitsprüfung zu erzeugen und/oder zu verändern.

7. Vorrichtung (1) nach Anspruch 1, wobei die Steuereinheit (8) dafür eingerichtet ist, Kalibrierdaten zu speichern und/oder Kalibrierdaten während der Empfindlichkeitsprüfung und/oder während der Erzeugung des Prüfungsergebnisses zu verarbeiten oder zu verwenden, wobei die genannten Kalibrierdaten während einer früheren Empfindlichkeitsprüfung bereitgestellt wurden.

8. Verfahren zum automatischen Prüfen der Temperaturempfindlichkeit eines Patienten, das die folgenden Schritte umfasst:
- Liefern einer Anzahl von Kontaktflächen (4, 5), die dafür eingerichtet sind, eine Temperaturstimulation einer Körperfläche (2, 3) des Patienten zu verursachen,
- Starten der Empfindlichkeitsprüfung,
- Empfangen einer Reaktion des Patienten in Bezug auf seine Empfindung,
- Erzeugen eines Reaktionssignals entsprechend der Reaktion des Patienten,
- automatisches Steuern der Empfindlichkeitsprüfung in Abhängigkeit vom Reaktionssignal, und
- Überwachen, ob der Patient die Prüfung korrekt durchführt und Erzeugen eines Einhaltungsfaktors.

9. Verfahren nach Anspruch 8, mit den Schritten des automatischen Erzeugens und/oder Veränderns der Empfindlichkeitsprüfung oder eines bestimmten Teils der Empfindlichkeitsprüfung in Abhängigkeit vom Reaktionssignal.

10. Verfahren nach Anspruch 8, mit dem Schritt des Erzeugens eines Prüfungsergebnisses ausgehend von den Reaktionssignalen.

11. Verfahren nach Anspruch 8, mit dem Schritt des Speicherns von Kalibrierdaten und/oder Verarbeitens oder Verwendens von Kalibrierdaten während der Empfindlichkeitsprüfung und/oder während der Erzeugung des Prüfungsergebnisses, wobei die genannten Kalibrierdaten während einer früheren Empfindlichkeitsprüfung bereitgestellt wurden.

12. Computerprogramm zum automatischen Prüfen der Temperaturempfindlichkeit eines Patienten, das Folgendes umfasst:
- Computerprogrammanweisungen zum Erzeugen eines Reaktionssignals in Übereinstimmung mit der Reaktion des Patienten auf eine Temperaturstimulation einer Körperfläche (2, 3) des Patienten,
- Computerprogrammanweisungen zum automatischen Steuern der Empfindlichkeitsprüfung in Abhängigkeit vom Reaktionssignal, und
- Computerprogrammanweisungen zum Erzeugen eines Einhaltungsfaktors, der angibt, ob der Patient die Prüfung ordnungsgemäß durchführt,
wenn das Computerprogramm in einem Computer (18) ausgeführt wird.

## Revendications

1. Dispositif (1) permettant de tester automatiquement la sensibilité à la température d'un patient, comprenant :
- un certain nombre de zones de contact (4, 5), chaque zone étant agencée de manière à entrer en contact avec une zone (2, 3) du corps du patient, et étant en outre à même d'amener une stimulation en température de cette zone du corps (2, 3);
- une unité de réaction (7) à même de recevoir une réaction du patient par rapport à la sensation du patient et en outre à même de générer un signal de réaction correspondant ;
- une unité de commande (8) à même de commander automatiquement le test de sensibilité en fonction du signal de réaction, et **caractérisé par**
un certain nombre de moyens de contrôle (16, 17) à même de contrôler si le patient exécute correctement ou non le test et en outre à même de générer un facteur de conformité qui peut être utilisé par l'unité de commande (8).

2. Dispositif (1) suivant la revendication 1, dans lequel les zones de contact (4, 5) sont thermo-conductrices et/ou sensibles à la pression.

3. Dispositif (1) suivant la revendication 1, dans lequel les zones de contact (4, 5) sont des pièces d'une balance.

4. Dispositif (1) suivant la revendication 1, dans lequel l'unité de réaction comprend un certain nombre d'éléments actionneurs (9, 10) et/ou un certain nombre d'éléments récepteurs sans contact (16).

5. Dispositif (1) suivant la revendication 1, dans lequel l'unité de réaction (7) est à même de générer un signal de réaction correspondant au fait que le patient fournit ou non une réaction dans un laps de temps défini et/ou correspondant au temps de réaction du patient.

6. Dispositif (1) suivant la revendication 5, dans lequel l'unité de commande (8) est à même de générer automatiquement et/ou de modifier le test de sensibilité ou une certaine partie du test de sensibilité, en fonction du signal de réaction.

7. Dispositif (1) suivant la revendication 1, dans lequel l'unité de commande (8) est à même de mémoriser des données d'étalonnage et/ou de traiter ou d'utiliser des données d'étalonnage pendant le test de sensibilité et/ou pendant la génération du résultat du test, lesdites données d'étalonnage étant fournies pendant un test de sensibilité antérieur.

8. Procédé permettant de tester automatiquement la sensibilité à la température d'un patient, comprenant les étapes suivantes :
- la mise à disposition d'un certain nombre de zones de contact (4, 5) étant à même d'amener une stimulation en température d'une zone du corps (2, 3) du patient;
- le démarrage du test de sensibilité ;
- la réception d'une réaction du patient par rapport à la sensation du patient ;
- la génération d'un signal de réaction en fonction de la réaction du patient ;
- la commande automatique du test de sensibilité en fonction du signal de réponse, et
- le fait de contrôler si le patient exécute correctement ou non le test et la génération d'un facteur de conformité.

9. Procédé suivant la revendication 8, comprenant les étapes de génération automatique et/ou de modification du test de sensibilité ou d'une certaine partie du test de sensibilité, en fonction du signal de réaction.

10. Procédé suivant la revendication 8, comprenant l'étape de génération d'un résultat de test sur la base des signaux de réaction.

11. Procédé suivant la revendication 8, comprenant l'étape de mémorisation de données d'étalonnage et/ou de traitement ou d'utilisation de données d'étalonnage pendant le test de sensibilité et/ou pendant la génération du résultat du test, lesdites données d'étalonnage étant fournies pendant un test de sensibilité antérieur.

12. Programme informatique permettant de tester automatiquement la sensibilité à la température d'un patient, comprenant :
- des instructions de programme informatique pour générer un signal de réaction en fonction de la réaction du patient à une stimulation en température d'une zone du corps (2, 3) du patient ;
- des instructions de programme informatique pour commander automatiquement le test de sensibilité en fonction du signal de réponse, et
- des instructions de programme informatique pour générer un facteur de conformité indiquant si le patient exécute correctement ou non le test,
lorsque le programme informatique est exécuté dans un ordinateur (18).
